# EUROPÄISCHE PATENTANMELDUNG

(11) **EP 1 129 698 A1**
(43) Veröffentlichungstag der Anmeldung: **05.09.2001**
(21) Anmeldenummer: 01103169.7
(22) Anmeldetag: 10.02.2001
(51) Int. Cl.: A61K 7/48

(54) **Wässrig-ethanolische Zubereitungen mit einem Gehalt an Chitosan**

(30) Priorität: 02.03.2000 DE 10010199
(71) Anmelder: Beiersdorf Aktiengesellschaft, 20245 Hamburg (DE)
(72) Erfinder: Schneider, Günther, Dr., 22607 Hamburg (DE); Kröpke, Rainer, 22869 Schenefeld (DE); Kaden, Waltraud, 25469 Halstenbek (DE); Winkler, Gabriele, 22880 Wedel (DE)

(57) **Zusammenfassung**

Kosmetische Gesichtswässer enthaltend
(a) Chitosan eines mittleren Molekulargewichtes von 10.000 bis 1.000.000 g/mol und eines Deacylierungsgrades von 10 bis 99%
(b) 10 - 80 Gew.-% Ethanol
(c) 0 - 5 Gew.-% an einem oder mehreren Lipiden,
(d) 0 - 5 Gew.-% an einem oder mehreren Fettalkoholen.

## Beschreibung

Die vorliegende Erfindung betrifft kosmetische und dermatologische Zubereitungen mit einem Gehalt an Chitosan

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum comeum), den für die Barrierefunktion bedeutenden Teil darstellt. Das heute in der Fachwelt anerkannte Hautmodell von Elias (*P. M. Elias*, *Structure and Function of the Stratum Comeum Permeability Barrier, Drug Dev. Res.* ***13****, 1988, 97-105*) beschreibt die Hornschicht als Zwei-Komponenten-System, ähnlich einer Ziegelsteinmauer (Ziegelstein-Mörtel-Modell). In diesem Modell entsprechen die Homzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Wäßrige alkoholische Lösungen stellen die Basis für sogenannte Tonics dar. Solche sind Gesichtswässer, die meist zum Abschluß der Gesichtspflege aufgelegt werden, um dem Gesicht ein Frischegefühl und ein erfrischtes Aussehen zu verleihen. Neben Parfum und anderen Zusätzen enthalten Tonics in der Regel Feuchthaltemittel wie beispielsweise Glycerin.

Üblicherweise liegt der Alkoholgehalt von Gesichtswässern im Bereich von 10 - 20 Gew.-%. Ein höherer Gehalt an Ethanol wäre bisweilen wünschenswert, um bestimmte Rezepturbestandteile, beispielsweise Parfumbestandteile, besser lösen zu können, andererseits führt erhöhter Ethanolgehalt nicht selten zu Hautreizung zumindest bei empfindlicher Haut.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P. Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel: 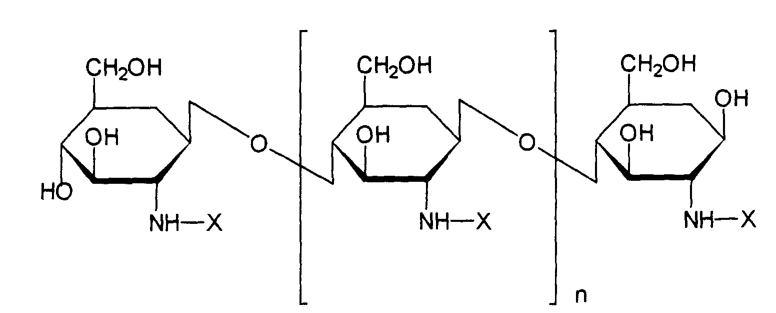 dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel 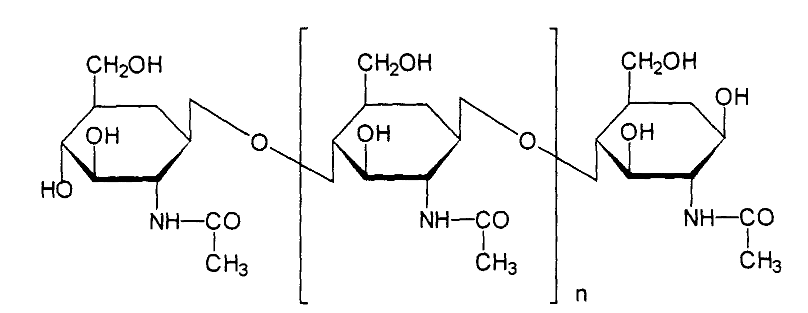 gekennzeichnet ist. Chitin ist wesentlicher Bestandteil des Ektoskeletts ['oχ 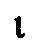 των = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wäßrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Die darin aufgeführten Emulsionen (z.B. mit dem O/W-Emulgator Cetylstearylglucosid im Gemisch mit Cetylstearylalkohol) haben jedoch hinsichtlich ihrer sensorischen Qualität gewisse Nachteile.

Aufgabe der Erfindung war es also, diesem Übelstande abzuhelfen und Gesichtswässer bzw. alkoholische Tonics zur Verfügung zu stellen, die einesteils, bestimmungsgemäß angewandt, dem Gesicht ein Frischegefühl und ein erfrischtes Aussehen verleihen, ohne aber formulierungstechnische Vorteile durch unangenehmes Hautgefühl, beispielsweise Klebrigkeit, oder gar verminderte Hautfreundlichkeit erkaufen zu müssen.

Erstaunlicherweise werden diese Aufgaben gelöst durch kosmetische Gesichtswässer enthaltend
(a) Chitosan eines mittleren Molekulargewichtes von 10.000 bis 1.000.000 g/mol und eines Deacylierungsgrades von 10 bis 99%
(b) 10 - 80 Gew.-% Ethanol
(c) 0 - 5 Gew.-% an einem oder mehreren Lipiden,
(d) 0 - 5 Gew.-% an einem oder mehreren Fettalkoholen.

Überraschend war, daß die erfindungsgemäßen Zubereitungen auch bei höheren Alkoholgehalten nicht hautreizend wirken, und daß selbst bei höheren Chitosangehalten kein klebriges Hautgefühl auftritt. Vielmehr sind erfindungsgemäß Gesichtswässer erhältlich, die ein seidiges Hautgefühl vermitteln und der Gesichtshaut Frischegefühl und frisches Aussehen verleihen.

Der oder die an sich fakultativ einzusetzenden Fettalkohole wird oder werden bevorzugt gewählt aus der Gruppe der verzweigten und unverzweigten Alkylalkohole mit 12 bis 40 Kohlenstoffatomen.

Bevorzugter, erfindungsgemäß verwendeter Fettalkohol ist der Cetyl-Stearylalkohol (ein Gemisch aus Hexadecanol-1 und Octadecanol-1 zu etwa gleichen Anteilen).

Die Gesamtmenge an einem oder mehreren erfindungsgemäß verwendeten Fettalkoholen in den fertigen kosmetischen oder dermatologischen Zubereitungen wird vorteilhaft aus dem Bereich von 0,1 - 5,0 Gew.-%, bevorzugt 0,5 -1,0 Gew.-% gewählt, bezogen auf das Gesamtgewicht der Zubereitungen.

Der oder die an sich fakultativ einzusetzenden Lipidkomponenten werden bevorzugt gewählt aus der Gruppe der üblichen kosmetischen Ölkomponenten.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölkomponenten können auch vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer können die Ölkomponenten vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft werden die Ölkomponenten gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft können die Ölkomponenten ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, besser Octamethylcyclotetrasiloxan, Polymethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Erfindungsgemäß bevorzugt sind Chitosane mit einem Deacetylierungsgrad > 25 %, insbesondere > 55 bis 99% % [bestimmt mittels ¹H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Erfindungsgemäß enthalten kosmetische Gesichtswässer 0,01 bis 10 Gew.-%, vorteilhaft 0,1 bis 5 Gew.-%, ganz besonders bevorzugt 0,25 bis 2,5 Gew.-% Chitosane.

Die Einarbeitung des Chitosans erfolgt in der Regel dadurch, daß Chitosan in Wasser mit Hilfe von organischen oder anorganischen Säuren auf einen pH-Wert von ca. 3,5 - 5,5 gestellt, wobei - in der Regel durch Rühren - eine Lösung des Chitosans erhalten wird. Die so erhältlichen klaren Lösungen zeichnen sich beispielsweise als 2 Gew. - %ige Lösung von Chitosan in 1,2% Milchsäure (90%ige wäßrige Lösung) durch eine Viskosität nach Viskotester-VT02 (Haake) aus, die im Bereich von 100 bis 10.000 mPas, vorzugsweise von 200 bis 5.000 mPas liegt.

Vorteilhaft kann beispielsweise Milchsäure verwendet werden, es ist aber ebenfalls von Vorteil, andere Säuren verwendet werden, die lösliche Chitosansalze ergeben, wie z.B. Phosphorsäure, Essigsäure, Ascorbinsäure (diese vorzugsweise unter Verwendung eines Schutzgases), Salzsäure, Glykolsäure, Salpetersäure, 2-Pyrrolidon-5-carbonsäure, Apfelsäure, Salicylsäure, Benzoesäure.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere anti-irritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Femer kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quatemäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z.B. Jaguar C 162 [Hydroxypropyl Guar Hydroxypropyltrimonium Chloride] bzw. modifizierten Magnesiumaluminiumsilikaten (z.B. Quatemium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Ebenfalls vorteilhaft ist, Zubereitungen gemäß der Erfindung amphotere bzw. zwitterionischen Tensiden (z.B. Cocoamidopropylbetain) und Moisturizern (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen.

Herstellungsbeispiel Chitosanlösung:

| | Gew.-% |
|---|---|
| Chitosan | 2,0 |
| Milchsäure (90%ige wäßrige Lösung) | 1,2 |
| Wasser | ad 100,00 |
| pH-Wert ca. | 4,5 |

Die Einarbeitung des Chitosans erfolgt dadurch, daß eine Suspension von micronisiertem Chitosan in Wasser mit der Milchsäure auf einen pH-Wert von 4,5 gestellt wird, wobei eine Lösung des Chitosans erhalten wird.

### Rezepturbeispiel 1

| | Gew.-% |
|---|---|
| Glycerin | 2,00 |
| Ethanol | 70,00 |
| Bisabolol | 0,10 |
| Panthenol | 1,50 |
| Chitosanlösung gem. Herstellungsbeispiel entsprechend einem Gesamtgehalt von Chitosan | 0,50 |
| Milchsäure | 0,33 |
| Niacinamid | 0,10 |
| Parfum | q.s. |
| Wasser | ad 100,00 |

Die Bestandteile (einschließlich der Chitosanlösung gemäß Herstellungsbeispiel) werden bei Raumtemperatur zusammengegeben und verrührt, bis eine klare Lösung entstanden ist.

### Rezepturbeispiel 2

| | Gew.-% |
|---|---|
| Glycerin | 2,00 |
| Ethanol | 70,00 |
| Bisabolol | 0,10 |
| Panthenol | 1,50 |
| Chitosanlösung gem. Herstellungsbeispiel entsprechend einem Gesamtgehalt von Chitosan | 0,50 |
| Milchsäure | 0,33 |
| Niacinamid | 0,10 |
| Na₃HEDTA | 0,20 |
| Parfum | q.s. |
| Wasser | ad 100,00 |

### Rezepturbeispiel 3

| | Gew.-% |
|---|---|
| Glycerin | 2,00 |
| Ethanol | 70,00 |
| Bisabolol | 0,10 |
| Panthenol | 1,50 |
| Chitosanlösung gem. Herstellungsbeispiel entsprechend einem Gesamtgehalt von Chitosan | 0,50 |
| Milchsäure | 0,33 |
| Niacinamid | 0,10 |
| Na₃HEDTA | 0,20 |
| Parfum | q.s. |
| Wasser | ad 100,00 |

### Rezepturbeispiel 4

| | Gew.-% |
|---|---|
| Glycerin | 2,00 |
| Ethanol | 70,00 |
| Bisabolol | 0,10 |
| Panthenol | 1,50 |
| Chitosanlösung gem. Herstellungsbeispiel entsprechend einem Gesamtgehalt von Chitosan | 0,50 |
| Milchsäure | 0,33 |
| Niacinamid | 0,10 |
| Na₃HEDTA | 0,20 |
| Verdicker | 0,10 |
| Parfum | q.s. |
| Wasser | ad 100,00 |

## Patentansprüche

1. Kosmetische Gesichtswässer enthaltend
(a) Chitosan eines mittleren Molekulargewichtes von 10.000 bis 1.000.000 g/mol und eines Deacylierungsgrades von 10 bis 99%
(b) 10 - 80 Gew.-% Ethanol
(c) 0 - 5 Gew.-% an einem oder mehreren Lipiden,
(d) 0 - 5 Gew.-% an einem oder mehreren Fettalkoholen.

2. Verwendung von Chitosan eines mittleren Molekulargewichtes von 10.000 bis 1.000.000 g/mol und eines Deacylierungsgrades von 10 bis 99 % zur Herstellung hautfreundlicher und/oder nichtklebriger alkoholischer kosmetischer Gesichtswässer.

3. Gesichtswässer nach Anspruch 1 oder Verwendung nach Anspruch 2, **dadurch gekennzeichnet**, daß die Gesamtmenge an einem oder mehreren Chitosanen aus dem Bereich von 0,01 bis 10 Gew.-%, vorteilhaft 0,1 bis 5 Gew.-%, ganz besonders bevorzugt 0,25 bis 2,5 Gew.-% gewählt wird, bezogen auf das Gesamtgewicht der Zubereitungen.

4. Gesichtswässer nach Anspruch 1 oder Verwendung nach Anspruch 2, **dadurch gekennzeichnet**, daß die Gesamtmenge an einem oder mehreren Fettalkoholen in den fertigen kosmetischen oder dermatologischen Zubereitungen aus dem Bereich von 0,1 - 5,0 Gew.-%, bevorzugt 0,5 -1,0 Gew.-% gewählt wird, bezogen auf das Gesamtgewicht der Zubereitungen.

5. Gesichtswässer nach Anspruch 1 oder Verwendung nach Anspruch 2, **dadurch gekennzeichnet**, daß die Gesamtmenge der Lipidkomponente oder der Lipidkomponenten, beispielsweise vorteilhaft eines oder mehrerer Phospholipide, insbesondere vorteilhaft eines oder mehrerer Lecithine, in den Zubereitungen aus dem Bereich von 0,001 bis 5 Gew.-% gewählt wird, besonders bevorzugt 0,05 - 2,5 Gew.-%, insbesondere 0,5 - 1 % Gew.-%, jeweils bezogen auf das Gesamtgewicht der Zubereitung.
